# EUROPEAN PATENT APPLICATION

(11) **EP 4 238 586 A1**
(43) Date of publication of application: **06.09.2023**
(21) Application number: 22186162.8
(22) Date of filing: 21.07.2022
(51) Int. Cl.: A61L 2/10, A61B 1/12, A61B 90/70, A61M 25/00

(54) **LASER DISINFECTING AND STERILIZING DEVICE FOR MEDICAL INTERVENTIONAL CATHETER**

(30) Priority: 01.03.2022 CN 202210198322
(71) Applicant: Guangdong Guozhi Photonics Technology Co., Ltd., Guangdong 523808 (CN)
(72) Inventor: ZHONG, Chen, Dongguan City, 523808 (CN); HU, Chang, Dongguan City, 523808 (CN)
(74) Representative: Wächter, Jochen

(57) **Abstract**

A laser disinfecting and sterilizing device for a medical interventional catheter is disclosed, comprising an ultraviolet laser device, a coupling module, a conductive optical fiber, and a traction device. The conductive optical fiber has an attenuation region for scattering ultraviolet laser from the conductive optical fiber to an inner wall of a catheter, and the attenuation region extends into the catheter. The traction device is connected to the conductive optical fiber and drives the conductive optical fiber to move back and forth in the catheter. The ultraviolet laser is emitted into the conductive optical fiber via the coupling module, the attenuation region on the conductive optical fiber scatters the ultraviolet laser into the catheter, and the traction device pulls the conductive optical fiber to move back and forth in the catheter, the emitted ultraviolet laser can completely disinfect and sterilize the inner wall of the catheter.

## Description

### TECHNICAL FIELD

The present disclosure relates to the technical field of catheter disinfection, and in particular relates to laser disinfecting and sterilizing device for a medical interventional catheter.

### BACKGROUND ART

Interventional therapy refers to that special catheters or devices are delivered to lesion areas in human body via the arteries, the veins, the natural ducts of the digestive system, bile ducts, or post-surgical drainage ducts of human body, so as to obtain information about histiocyte, bacteria, or biochemical aspect for the purpose of diagnosing disease, as well as for various special therapy. Interventional therapy is widely used in clinical therapy for its advantages such as minimally invasive, accurate localization, rapid efficacy, and less side effects and complications. Catheters used in the interventional therapy, such as endoscopes, are reusable, therefore the catheters need to be disinfected and sterilized after each surgery.

Cleaning and sterilization of the outer walls of the catheters is easy to achieve, while cleaning and sterilization of the inner walls of the catheters is more difficult, especially for thin and long catheters. At present, cleaning and disinfection of the catheters is mainly achieved by manually flushing the inner walls of the catheters with saline or disinfectant aspirated by syringes. Such cleaning way is low in efficiency and incomplete in cleaning. There is also a way of cleaning the inner walls of the catheters by using spray guns. The cleaning fluid is injected into the catheters at high pressure for flushing and disinfection; however, such cleaning way is not suitable for thin catheters. There is a further way of soaking the catheters into the disinfectant for sterilization; however, for long catheters, an inner cavity cannot be filled with the disinfectant, resulting in incomplete disinfection; and it is difficult to completely disinfect and sterilize the inner walls of the catheters even with the assistance of water pumps. Moreover, it is easy to cause cross infection of patients as the above disinfecting and sterilizing ways are extremely low in efficiency and incomplete in disinfection and sterilization. Therefore, it is necessary to design a medical catheter disinfecting and cleaning device to modify the problems above.

### SUMMARY

A laser disinfecting and sterilizing device for a medical interventional catheter is provided by the present disclosure , with an objective of solving the problem of incomplete disinfection and sterilization of medical interventional catheters. To achieve the objective described above, the present disclosure provides the following solutions:

A laser disinfecting and sterilizing device for a medical interventional catheter comprising an ultraviolet laser device, a coupling module, a conductive optical fiber, and a traction device, wherein the coupling module is located adjacent to the light-emitting end of the ultraviolet laser device, ultraviolet laser emitted from the ultraviolet laser device is emitted into the coupling module, the conductive optical fiber is connected to the coupling module, and the ultraviolet laser is coupled into the conductive optical fiber, the conductive optical fiber has an attenuation region for scattering the ultraviolet laser from the conductive optical fiber to the inner wall of a catheter, and the attenuation region extends into the catheter, the traction device is connected to the conductive optical fiber and drives the conductive optical fiber to move back and forth in the catheter.

In an embodiment, the ultraviolet laser can have a wavelength range from 201 to 350 nm.

In an embodiment, the conductive optical fiber can be connected to the coupling module by an optical fiber quick connector.

In an embodiment, the traction device can be provided with a traction head, the traction head is connected to the conductive optical fiber, and the traction head is provided with a brush head for cleaning the inner wall of the catheter.

In an embodiment, the conductive optical fiber can comprise a fiber core, a cladding layer wrapped on the outside the fiber core, and a coating layer wrapped on the outside the cladding layer, the conductive optical fiber sequentially comprises an input end, the attenuation region and an output end in the length direction of the conductive optical fiber, the coating layer is wrapped on the outside the cladding layer at both the input end and the output end, and the cladding layer on the attenuation region is provided with light leakage structures.

In an embodiment, the light leakage structures can be non-continuous annular grooves, continuous helical grooves, or a plurality of slots.

In an embodiment, the light leakage structures on the conductive optical fiber can be uniformly distributed at equal intervals or non-uniformly distributed.

In an embodiment, the light leakage structures can be processed by using a chemical corrosion method or a laser engraving method.

In an embodiment, the coupling module can be internally provided with a first lens and a second lens that is separated from the first lens, the ultraviolet laser emitted into the coupling module is collimated by the first lens and then is emitted to the second lens, and then the ultraviolet laser is coupled by the second lens and then is emitted into the conductive optical fiber.

In accordance with specific embodiments provided by the present disclosure, the present disclosure has the following technical effects: the ultraviolet laser emitted from the ultraviolet laser device is emitted into the conductive optical fiber via the coupling module, the attenuation region on the conductive optical fiber scatters the ultraviolet laser into the catheter, and the traction device pulls the conductive optical fiber to move back and forth in the catheter, such that the emitted ultraviolet laser can completely disinfect and sterilize the inner wall of the catheter. Compared with a traditional catheter disinfecting method, the device is high in disinfection efficiency, complete in disinfection and sterilization, and excellent in disinfecting and sterilizing effect. The whole device has simple structure, can be applied to medical catheters with different sizes, and is simple to operate and low in maintenance cost.

### BRIEF DESCRIPTION OF THE DRAWINGS

To describe the technical solutions in the embodiments of the present disclosure or in the prior art more clearly, the following briefly introduces the accompanying drawings required for describing the embodiments. Apparently, the accompanying drawings described in the following show merely some embodiments of the present disclosure, and a person of ordinary skill in the art may still derive other drawings from these accompanying drawings without creative efforts.

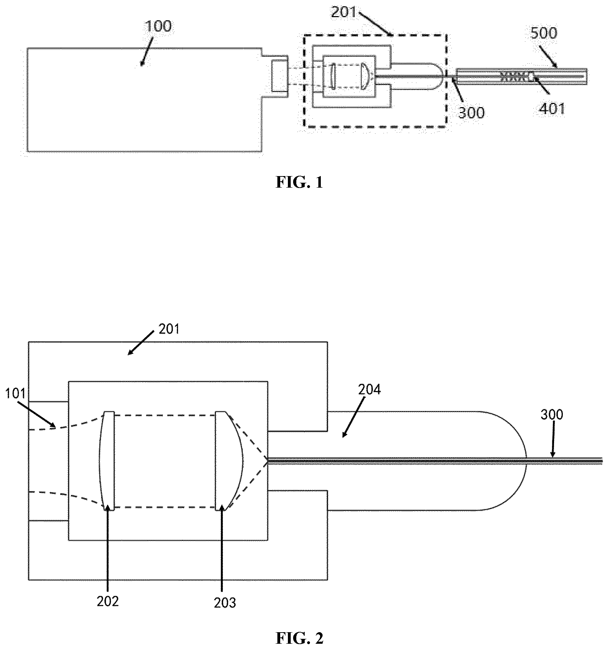

FIG. 1 is a structural diagram of a laser disinfecting and sterilizing device for a medical interventional catheter in accordance with the present disclosure;
FIG. 2 is an assembly schematic diagram of a coupling module and a conductive optical fiber of a laser disinfecting and sterilizing device for a medical interventional catheter in accordance with the present disclosure;
FIG. 3 is a structural schematic diagram of an annular attenuation region of a conductive optical fiber of a laser disinfecting and sterilizing device for a medical interventional catheter in accordance with the present disclosure;
FIG. 4 is a structural schematic diagram of a helical attenuation region of a conductive optical fiber of a laser disinfecting and sterilizing device for a medical interventional catheter in accordance with the present disclosure;
FIG. 5 is a structural schematic diagram of a hole-shaped attenuation region of a conductive optical fiber of a laser disinfecting and sterilizing device for a medical interventional catheter in accordance with the present disclosure;
FIG. 6 is a schematic diagram of the scattering of an attenuation region of a conductive optical fiber of a laser disinfecting and sterilizing device for a medical interventional catheter in accordance with the present disclosure;
FIG. 7 is a schematic diagram of a disinfecting and sterilizing process inside a catheter of a laser disinfecting and sterilizing device for a medical interventional catheter in accordance with the present disclosure.

### DETAILED DESCRIPTION OF THE EMBODIMENTS

An objective of the present disclosure is to provide a laser disinfecting and sterilizing device for a medical interventional catheter to provide a disinfecting and sterilizing device suitable for inner walls of the catheters with excellent disinfecting and sterilizing effect.

To make the objectives, the features and the advantages of the present disclosure more apparent and understandable, the following further describes the present disclosure in detail with reference to the accompanying drawings and specific embodiments.

A laser disinfecting and sterilizing device for a medical interventional catheter comprising an ultraviolet laser device 100, a coupling module 201, a conductive optical fiber 300, and a traction device 400. The ultraviolet laser device 100 emits high-energy ultraviolet laser 101. Alternatively, the ultraviolet laser device 100 may be a solid ultraviolet laser device, a gas ultraviolet laser device, or a semiconductor ultraviolet laser device.

The coupling module 201 is located adjacent to the light-emitting end of the ultraviolet laser device 100, the axis of the light passing hole of the coupling module 201 is collinear with the axis of the light outlet of the ultraviolet laser 100, the ultraviolet laser 101 emitted from the ultraviolet laser device 100 is emitted into the coupling module 201, and the ultraviolet laser 101 is coupled into the conductive optical fiber 300 by the coupling module 201. The conductive optical fiber 300 is connected to the coupling module 20, and the ultraviolet laser 101 is coupled into the conductive optical fiber 300. The conductive optical fiber 300 has an attenuation region 310 for scattering the ultraviolet laser 101 from the conductive optical fiber 300 to an inner wall of a catheter 500, and the attenuation region 301 extends into the catheter 500. The traction device 400 is connected to the conductive optical fiber 300 and drives the conductive optical fiber 300 to move back and forth in the catheter 500.

As the ultraviolet laser 101 emitted from the ultraviolet laser device 100 enters the conductive optical fiber 300 via the coupling module 201, the attenuation region 301 on the conductive optical fiber 300 scatters the ultraviolet laser 101 into the catheter 500, and the traction device 400 pulls the conductive optical fiber 300 to move back and forth in the catheter 500, the emitted ultraviolet laser 101 can completely disinfect and sterilize the inner wall of the catheter. Compared with a traditional catheter disinfecting method, the device provided by the present disclosure is high in disinfection efficiency, complete in disinfection and sterilization, and excellent in disinfecting and sterilizing effect. The whole device has a simple structure, can be applied to medical catheters with different sizes, and is simple in operation and low in maintenance cost.

In an embodiment, the ultraviolet laser 101 has a wavelength range from 201 to 350 nm which has an excellent disinfecting and sterilizing effect.

In an embodiment, the conductive optical fiber 300 is connected to the coupling module 201 by an optical fiber quick connector 204. The optical fiber quick connector 204 is able to ensure the rapid alignment of the conductive optical fiber 300 while for the different sizes of catheters, the replacement of conductive optical fibers with different sizes is convenient.

In an embodiment, the traction device 400 is provided with a traction head 401, the traction head 401 is connected to the conductive optical fiber 300, and the traction head 401 is provided with a brush head 402 for cleaning the inner wall of the catheter. In the process that the traction device 400 pulls the conductive optical fiber 300 to move, the brush head 402 abuts against the inner wall of the catheter 500, i.e. the inner wall of the catheter can be cleaned while it is disinfected and sterilized.

Illustratively, the traction head 401 may be regarded as a connection block, the traction device 400 may be a traction wire (steel wire), and the tip of the conductive optical fiber 300 and the traction wire both are tied to the connection block.

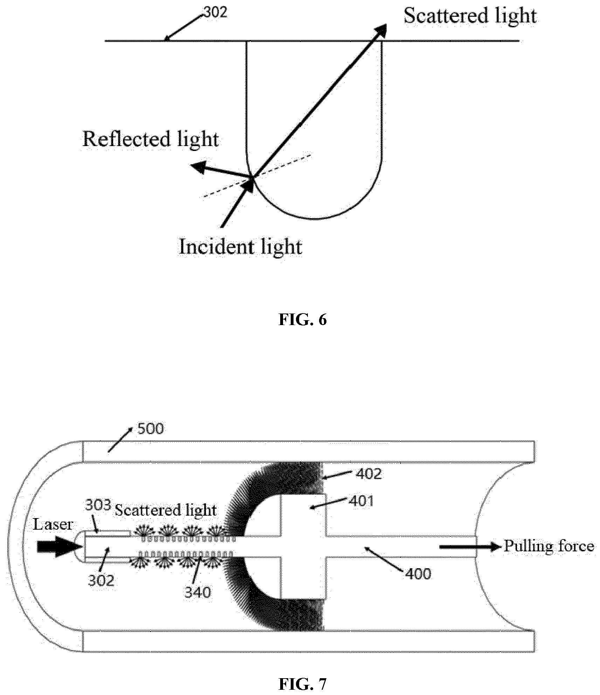

In an embodiment, the conductive optical fiber 300 comprises a fiber core 301, a cladding layer 302 wrapped on the outside the fiber core, and a coating layer 303 wrapped on the outside the cladding layer. The conductive optical fiber 300 sequentially comprises an input end 320, the attenuation region 310, and an output end 330 in the length direction of the conductive optical fiber 300. The coating layer 303 is wrapped on the outside the cladding layer 302 at the input end 320 and the output end 330. The cladding layer 302 on the attenuation region 310 is provided with light leakage structures 340, and the fiber core 301 is exposed from the light leakage structures 340. As shown in FIG. 6, the ultraviolet laser 101 conducted from the inside of the conductive optical fiber 300 is scattered out through the light leakage structures 340 on the attenuation region 310, and finally irradiated to the inner wall of the catheter 500 to achieve disinfection and sterilization.

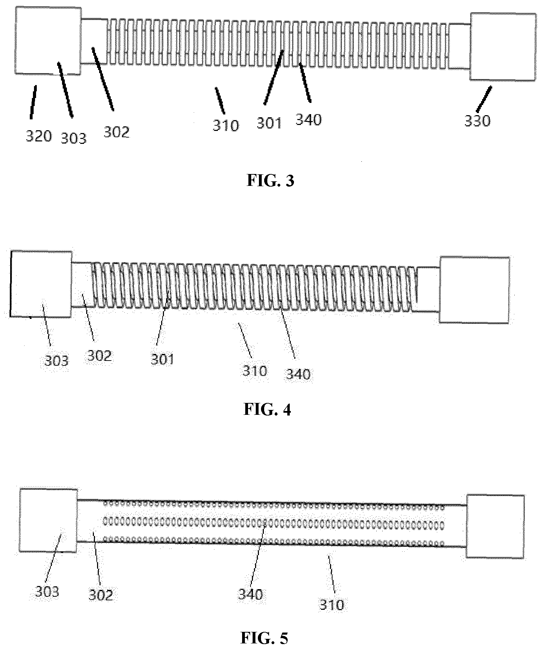

As shown in FIG. 3, in an embodiment, the light leakage structures 340 are non-continuous annular grooves, i.e. it can be appreciated that the light leakage structures 340 remove part of the cladding layer 302 to damage the total reflection of the laser inside the optical fiber to achieve scattering of the laser. As shown in FIG. 4, the light leakage structures 340 may also be continuous helical grooves. As shown in FIG. 5, the light leakage structures 340 may also be a plurality of slots which are arranged at intervals. It needs to be noted that the light leakage structures 340 may be any structures capable of meeting the demands of the present disclosure, which is not limited by the embodiments of the present disclosure.

In an embodiment, the light leakage structures on the conductive optical fiber 300 are uniformly distributed at equal intervals or non-uniformly distributed, which can be arranged based on different requirements of use.

In an embodiment, the light leakage structures are processed by using a chemical corrosion method or a laser engraving method.

In an embodiment, the coupling module 201 is internally provided with a first lens 202 and a second lens 203 that is separated from the first lens 202, the ultraviolet laser 101 emitted into the coupling module 201 is collimated by the first lens 202 and then is emitted to the second lens 203, and the ultraviolet laser 101 is coupled by the second lens 203 and then is emitted into the conductive optical fiber 300.

Specific examples are used for illustration of the principles and implementation methods of the present disclosure. The description of the embodiments is merely used to help illustrate the method and its fiber core principles of the present disclosure. The described embodiments are merely a part rather than all of the embodiments of the present disclosure. All other embodiments obtained by a person of ordinary skill in the art based on the embodiments of the present invention without creative efforts shall fall within the scope of the present disclosure.

## Claims

1. A laser disinfecting and sterilizing device for a medical interventional catheter, comprising an ultraviolet laser device, a coupling module, a conductive optical fiber, and a traction device,
wherein the coupling module is located adjacent to a light-emitting end of the ultraviolet laser device,
ultraviolet laser emitted from the ultraviolet laser device is emitted into the coupling module,
the conductive optical fiber is connected to the coupling module, and the ultraviolet laser is coupled into the conductive optical fiber,
the conductive optical fiber has an attenuation region for scattering the ultraviolet laser from the conductive optical fiber to an inner wall of a catheter, and the attenuation region extends into the catheter,
the traction device is connected to the conductive optical fiber and drives the conductive optical fiber to move back and forth in the catheter.

2. The laser disinfecting and sterilizing device for a medical interventional catheter according to claim 1, wherein the ultraviolet laser has a wavelength range from 201 to 350 nm.

3. The laser disinfecting and sterilizing device for a medical interventional catheter according to claim 1, wherein the conductive optical fiber is connected to the coupling module by an optical fiber quick connector.

4. The laser disinfecting and sterilizing device for a medical interventional catheter according to claim 1, wherein the traction device is provided with a traction head, the traction head is connected to the conductive optical fiber, and the traction head is provided with a brush head for cleaning the inner wall of the catheter.

5. The laser disinfecting and sterilizing device for a medical interventional catheter according to claim 1, wherein the conductive optical fiber comprises a fiber core, a cladding layer wrapped on an outside the fiber core, and a coating layer wrapped on an outside the cladding layer, the conductive optical fiber sequentially comprises an input end, the attenuation region and an output end in a length direction of the conductive optical fiber, the coating layer is wrapped on the outside the cladding layer at both the input end and the output end, and the cladding layer on the attenuation region is provided with light leakage structures.

6. The laser disinfecting and sterilizing device for a medical interventional catheter according to claim 5, wherein the light leakage structures are non-continuous annular grooves, continuous helical grooves, or a plurality of slots.

7. The laser disinfecting and sterilizing device for a medical interventional catheter according to claim 6, wherein the light leakage structures on the conductive optical fiber are uniformly distributed at equal intervals or non-uniformly distributed.

8. The laser disinfecting and sterilizing device for a medical interventional catheter according to claim 5, wherein the light leakage structures are processed by using a chemical corrosion method or a laser engraving method.

9. The laser disinfecting and sterilizing device for a medical interventional catheter according to claim 1, wherein the coupling module is internally provided with a first lens and a second lens that is separated from the first lens, the ultraviolet laser emitted into the coupling module is collimated by the first lens and then is emitted to the second lens, and the ultraviolet laser is coupled by the second lens and then is emitted into the conductive optical fiber.
